(19) Europäisches Patentamt
European Patent Office
Office européen des brevets

(11) **EP 1 605 926 B1**

(12) **EUROPEAN PATENT SPECIFICATION**

(45) Date of publication and mention
of the grant of the patent:
**14.03.2012 Bulletin 2012/11**

(51) Int Cl.:
*A61K 36/48* (2006.01)   *A61K 36/483* (2006.01)
*A61K 36/15* (2006.01)   *A61K 36/72* (2006.01)
*A61K 31/075* (2006.01)   *A61P 1/16* (2006.01)

(21) Application number: **04723853.0**

(22) Date of filing: **26.03.2004**

(86) International application number:
**PCT/KR2004/000689**

(87) International publication number:
**WO 2004/084875 (07.10.2004 Gazette 2004/41)**

(54) **Use of pinitol for treatment of liver diseases in mammals**

Verwendung von Pinitol zur Behandlung von Leberkrankheiten in Säugetieren

Utilisation de pinitol pour le traitement de maladie du foie chez les mammifères

(84) Designated Contracting States:
**AT BE BG CH CY CZ DE DK EE ES FI FR GB GR HU IE IT LI LU MC NL PL PT RO SE SI SK TR**

(30) Priority: **26.03.2003 KR 2003019018**

(43) Date of publication of application:
**21.12.2005 Bulletin 2005/51**

(73) Proprietor: **Amicogen, Inc.**
**Jinju-si,**
**Kyungsangnam-do 660-852 (KR)**

(72) Inventors:
• **SHIN, Yong Chul**
**Jinju-si, Kyungsangnam-do 660-773 (KR)**
• **JEON, Yeong Joong**
**Seoul 134-070 (KR)**
• **KIM, Jong Jin**
**Keumsan-myun**
**Jinju-si**
**Kyungsangnam-do 66 (KR)**

(74) Representative: **Goddar, Heinz J.**
**Forrester & Boehmert**
**Pettenkoferstrasse 20-22**
**80336 München (DE)**

(56) References cited:
**WO-A1-92/00744      WO-A1-02/060463**
**WO-A2-03/061553      US-A- 5 550 166**

• **DATABASE TCM [Online] SIPO; 1 April 1998 (1998-04-01), LAI CHUNRONG: "Oral liquid for treating tumor and liver diseases and process for preparation thereof" XP002520714 Database accession no. CN-96109862-A & CN 1 177 485 A (LAI CHUNRONG [CN]) 1 April 1998 (1998-04-01)**
• **DATABASE EPODOC EUROPEAN PATENT OFFICE, THE HAGUE, NL; "Agent for treatment and prevention of hepatitis" XP002520715 & JP 09 301877 A (YOUSHINDOU KK; TOA WOOL SPINNING & WEAVING CO) 25 November 1997 (1997-11-25)**
• **DATABASE WPI Week 198836 Thomson Scientific, London, GB; AN 1989-258968 XP002520716 & JP 01 186819 A (OTOMO F) 26 July 1989 (1989-07-26)**
• **HASE K ET AL: "Hepatoprotective effect of hovenia dulcis THUNB. on experimental liver injuries induced by carbon tetrachloride or D-galactosamine/lipopolys accharide" BIOLOGICAL & PHARMACEUTICAL BULLETIN (OF JAPAN), PHARMACEUTICAL SOCIETY OF JAPAN, TOKYO, JP, vol. 20, no. 4, 1 April 1997 (1997-04-01), pages 381-385, XP002992805 ISSN: 0918-6158**

EP 1 605 926 B1

- YOSHIKAWA M ET AL: "BIOACTIVE CONSTITUENTS OF CHINESE NATURAL MEDICINES. III. ABSOLUTE STEREOSTRUCTURES OF NEW DIHYDROVLAVONOLS, HOVENITINS I, II, AND III, ISOLATED FROM HOVENIAE SEMEN SEU FRUCTUS, THE SEED AND FRUIT OF HOVENIA DULCIS THUNB. (RHAMNACEAE): INHIBITORY EFFECT" YAKUGAKU ZASSHI - JOURNAL OF THE PHARMACEUTICAL SOCIETY OF JAPAN, JP, vol. 117, no. 2, 1 February 1997 (1997-02-01), pages 108-118, XP001223536 ISSN: 0031-6903
- SINGH R.K. ET AL.: 'Anti-inflammatory effect of (+)-pinitol' FITOTERAPIA vol. 72, no. 2, 2001, pages 169 - 170, XP008102762
- BATES S.H. ET AL.: 'Insuline-like effect of pinitol' BRITISH JOURNAL OF PHARMACOLOGY vol. 130, no. 8, 2000, pages 1944 - 1948, XP008101996
- DREYER D.L. ET AL.: 'Pinitol, a larval growth inhibitor for Heliothis zea in soybeans' EXPERIENTIA vol. 35, no. 9, 1979, pages 1182 - 1183, XP008103577

**Description**

**Field of the Invention**

The present invention relates to a use of pinitol ($C_7H_{14}O_6$, MW 194.18), or an extract of a plant containing pinitol for use in a method of preventing or treating a liver disease.

**Background of the invention**

The number of the population afflicted by various types of liver diseases have recently been on the increase due to dietary changes, stress, excessive intake of alcohol, and/or hepatotoxic substances. Liver cirrhoisis, in particular, which is caused by alcohol, drug, chemicals, metabolic diseases such as viral hepatitis and biliary disease, or autoimmunity diseases, suppress the liver function by lowering both the hepatic blood flow and metabolic enzyme activity and by changes in proteins in the blood and bile flow.

The hepatic function deteriorates and may develop into hepatitis, hepatocirrhosis or hepatic cancer as a result of excessive intake of alcohol or foods having a high lipid content, or infection by hepatitis B or C virus. In particular, the excessive intake of fat-containing foods and alcohol causes fatty liver leading to elevated levels of serum GOT (glutamate-oxaloacetate transaminase), OPT (glutamate-pyruvate transaminase) and γ-GTP (γ-glutamyl transpeptidase).

Oxidative stress also plays an important role in the attack by alcoholic liver-related diseases, non-alcoholic fatty liver-related diseases and viral liver-related diseases (Arteel GE, Gastroenterology, 2003, 124: 778-90; Loguercio C and Federico A. Free Radic. Biol. Med., 2003, 1; 34(1): 1-10; Mehta K et al., Nutr. Rev., 2002, 60(9): 289-93; Gebhardt R. Planta Med., 2002, 68(4): 289-96; Adachi M et al., Free Radic. Biol. Med., 2002, 15; 32(6): 487-91; Parola M et al., J. Hepatol., 2001, 35(2): 297-306). Superoxide dismutase (SOD), an anti-oxidation enzyme, participates in the treating or preventing liver-related diseases by way of mitigating the oxidative stress. It has also been reported that glutathione plays an important role as a non-enzymatic anti-oxidant in the protection of cells from the demage by radicals and also in the synthesis of proteins or DNA, material transportation and enzyme reactions.

Pinitol, which is metabolized into chiroinositol in the body, has been reported to be effective in treating or preventing fatness, hyperlipidemia and hypertension (USP No. 5,550,166). However, pharmacological activity of pinitol or chiroinositol in preventing or treating liver-related diseases has never been explored.

**Summary of the Invention**

Accordingly, it is an object of the present invention to provide a pharmacologically active substance for preventing and treating liver-related diseases by protecting the liver.

**Detailed Description of the Invention**

In accordance with one aspect of the present invention, there is provided a use of pinitol according to claim 1.

In accordance with one aspect of the present invention, there is provided a use of an extract of a plant containing pinitol according to claim 6.

The plant which may be used in the present invention is inclusive of soybean, pine, *Hovenia dulcis* Thunb, *Acanthopanax senticosus,* carob and the like, and preferably soybean and carob.

The extract of a plant containing pinitol or chiroinositol of the present invention can be prepared using such a solvent as water or an organic solvent, e.g., a lower alcohol, acetone, chloroform, methylenechloride, ether, ethylacetate, hexane and a mixture thereof. Examples of the lower alcohol are methanol, ethanol, propanol and butanol, preferably ethanol.

The plant used in the extraction procedure of the present invention may be of a dried powder form. Specifically, a water extract of a plant can be prepared by adding 5 to 15 fold volume of water, preferably 10-fold volume of water to a dried plant powder, extracting for 1 to 24 hours, preferably 2 to 5 hours at 10 to 80 °C, preferably 30 to 50°C, and then filtering. Alternately, 1 to 15-fold volume, preferably 10-fold volume of an organic solvent may be used to extract a plant powder at room temperature, to obtain an organic solvent extract. The above extraction procedure may be repeated two more times as needed. Also, after the filtration, a powder form of the extract can be prepared by removing the solvent under a reduced pressure.

In order to prevent and treat liver-related diseases, or to protect liver, pinitol or an extract of a plant containing pinitol can be administered to a mammal in the form of a composition containing, e.g., a pharmaceutical composition, a food composition or a beverage composition.

The content of pinitol in the pharmaceutical composition of the present invention may range form 10 to 100 wt%, preferably 5 to 50 wt% based on the total weight of the composition, and the amount of the plant extract containing pinitol in the pharmaceutical composition of the present invention may range form 1 to 50 wt%, preferably 5 to 30 wt% based on the total weight of the composition.

The pharmaceutical composition of the present invention can effectively protect the liver by way of reducing the levels of GOT, GPT and γ GTP in the blood and promoting the superoxide dismutase (SOD) activity and by way of increasing the glutathione level in the liver.

In spite of such potent efficacies, the inventive pharmaceutical composition containing pinitol or an extract of a plant containing pinitol shows little toxicity or mitogenicity in animal tests and exerts no adverse effects on the liver function.

A pharmaceutical formulation may be prepared in accordance with any of the conventional procedures. In preparing the formulation, the active ingredient is preferably admixed or diluted with a carrier, or enclosed within a carrier, sachet or other container. When the carrier serves as a diluent, it may be a solid, semi-solid or liquid material acting as a vehicle, excipient or medium for the active ingredient. Thus, the formulations may be in the form of a tablet, pill, powder, sachet, elixir, suspension, emulsion, solution, syrup, aerosol, soft and hard gelatin capsule, sterile injectable solution, sterile packaged powder and the like.

Examples of suitable carriers, excipients, and diluents are lactose, dextrose, sucrose, sorbitol, mannitol, calcium silicate, cellulose, methyl cellulose, microcrystalline cellulose, polyvinylpyrrolidone, water, methylhydroxybenzoates, propylhydroxybenzoates, talc, magnesium stearate and mineral oil. The formulations may additionally include fillers, anti-agglutinating agents, lubricating agents, wetting agents, flavoring agents, emulsifiers, preservatives and the like. The compositions of the invention may be formulated so as to provide quick, sustained or delayed release of the active ingredient after their administration to a mammal by employing any of the procedures well known in the art.

The pharmaceutical composition of the present invention can be administered via various routes including oral, transdermal, subcutaneous, intravenous and intramuscular introduction. In case of human, a typical daily dose of pinitol or chiroinositol may range from about 0.1 to 100 mg/kg body weight, preferably 1 to 50 mg/kg body weight, and can be administered in a single dose or in divided doses. However, it should be understood that the amount of the active ingredient actually administered ought to be determined in light of various relevant factors including the condition to be treated, the chosen route of administration, the age, sex and body weight of the individual patient, and the severity of the patient's symptom; and, therefore, the above dose should not be intended to limit the scope of the invention in any way.

Also disclosed is a method for preventing or treating liver-related diseases in mammals, which comprises administering thereto an effective amount of pinitol or chiroinositol or the extract of plant containing pinitol or chiroinositol.

Moreover, pinitol or the extract of plant containing pinitol can be incorporated in foods or beverages, as an additive or a dietary supplement, for the purpose of protecting liver. In this case, the content of pinitol in a food or beverage may range from 0.1 to 50 wt%, preferably 1 to 10 wt% based on the total weight of the food, and 0.01 to 10 g, preferably 0.1 to 1 g of per 100 mℓ of the beverage.

The health care beverage composition of the present invention may contain other components, e.g., deodorants and natural carbohydrates as in conventional beverages. As the deodorant, a natural deodorant such as taumatin, Stevia extract, e.g., levaudioside A, glycyrrhizin and the like, or a synthetic deodorant such as saccharin and aspartam may be used. Examples of such natural carbohydrates are monosaccharides such as glucose and fructose; disaccharides such as maltose and sucrose; conventional polysaccharides such as dextrin and cyclodextrin; and sugar alcohols such as xylitol, sorbitol and erythritol. The amount of the above-described natural carbohydrate is generally in the range of about 1 to 20 g, preferably 5 to 12 g based on 100 ml of beverage.

Other components that may be added to the inventive food or beverage composition are various nutrients, vitamins, minerals, synthetic flavoring agents, coloring agents, pectic acid and its salt, alginic acid and its salt, organic acids, protective colloidal adhesives, pH controlling agents, stabilizers, preservatives, glycerin, alcohol, carbonizing agents used in carbonated beverage. The amount of the above-described additives is generally in the range of about 0 to 20 weight portions based on 100 weight portions of the composition.

Moreover, the foods containing pinitol or the extract of plant containing pinitol or chiroinositol and the additional herbal extracts to develop health supplementary food, may include various foods, various beverages, various gums, vitamin complexes.

The following examples are intended to further illustrate the present invention.

Example 1: Preparation and Analysis of Plant Extract containing Pinitol

Soybean, pine needle, *Hovenia dulcis* Thunb, *Acanthopanax senticosus* were each dried and pulverized at room temperature and 10 g of the dried powder was extracted with 100 ml of distilled water at 25 °C for 6 hours.

The pinitol content of each extract was measured by High Performance Liquid Chromatography using Dionex Carbopak MA-1 column (eluent: 10 mM NaOH) and the result is shown in Table I.

**Table I**

| Plant | Pinitol content (g/kg) |
|---|---|
| Soybean | 4.4 |
| Pine needle | 6.7 |
| *Hovenia dulcis* Thunb | 4.0 |
| *Acanthopanax senticosus* | 4.8 |

Example 2: Toxicity of Orally Administered Pinitol

6 week-old, specific pathogen-free Sprague-Dawley female rats (15 heads), each weighing about 130 to 147 g, and male rats (15 heads), each weighing about 110 to 123 g, were bred under the condition of 23±3°C, 55±15 % relative humidity and 12L/12D photoperiod. Fodder (Harlan, U.S.A.) and water were sterilized and fed to the rats. The rats were acclimated for 1 week before the administration of pinitol.

Pinitol was dissolved in physiological saline and the solution was orally administered to each rat in an amount of 5,000 mg/kg of rat body weight. The solution was administered once and the rats were observed for 14 days for signs of adverse effects or death according to the following schedule: every hour for 6 hours after the administration and, every day thereafter. The weight changes of the rats were recorded at day 1, 3, 7 and 14 to examine the effect of pinitol. Further, on day 14, the rats were sacrificed and the internal organs were visually examined.

All the rats were alive at day 14 and pinitol showed no toxicity at a dose of 5,000 mg/kg. The autopsy revealed that the rats did not develop any pathological abnormality, and no weight loss was observed during the 14 day test period. Accordingly, it was concluded that pinitol is not toxic when orally administered to an animal.

Example 3: Protective Activity for the liver damaged by carbon tetrachloride

Sprague-Dawley rats (80 heads), each weighing about 180 to 200 g, were bred under the condition of temperature 23±3°C, 55±15 % relative humidity and 12L/12D photoperiod. Fodder (Harlan, U.S.A.) and water were sterilized and fed to the rats.

The rats were divided into 8 groups and carbon tetrachloride was injected subcutaneously into the rats except the rats of the normal group in an amount of 0.5 ml/kg at 1st and 5th day. On day 2, pinitol or chiroinositol dissolved in 10 ml of water was orally administered to the rats of the experimental groups in an amount of 5-20 mg/kg of rat body weight. The normal and control groups were treated with 10 ml of distilled water instead of pinitol. On day 8, GOT and GPT concentrations in the blood sample taken from the orbital vein of each rat was measured by using blood analyzer (Vitros DT-60, Johnson & Johnson) and the result is shown in Table II. The degree of inhibition (%) was then calculated in accordance with the following equation:

$$\text{Degree of Inhibition (\%)} = 100 \times \left[ 1 - \frac{\text{Experimental group} - \text{Normal group}}{\text{Control group} - \text{Normal group}} \right]$$

Table II

| | GOT | | GPT | |
|---|---|---|---|---|
| | IU/L | Inhibition (%) | IU/L | Inhibition (%) |
| Normal group | 65±8 | | 30±7 | |
| Control group | 522±48 | | 259±22 | |
| Experimental group 1 (Pinitol 5 mg/kg) | 298±28 | 49.7 | 134±7 | 54.6 |
| Experimental group 2 (pinitol 10 mg/kg) | 188±22 | 73.4 | 92±5 | 72.9 |
| Experimental group 3 (Pinitol 20 mg/kg) | 192±17 | 72.6 | 75±4 | 80.3 |

(continued)

|  | GOT | | GPT | |
|---|---|---|---|---|
|  | IU/L | Inhibition (%) | IU/L | Inhibition (%) |
| Experimental group 4 (chiroinositol 5 mg/kg) | 321±35 | 44.0 | 156±12 | 45.0 |
| Experimental group 5 (chiroinositol 10 mg/kg) | 205±28 | 69.4 | 80±9 | 78.2 |
| Experimental group 6 (chiroinositol 20 mg/kg) | 174±26 | 76.1 | 88±7 | 74.7 |

As can be seen from Table II, the control group rats showed markedly higher GOT and GPT concentrations than those of the normal group. The GOT and OPT concentrations of the pinitol-fed rats group were lower than those of the control group by 49.7 ~ 73.45% and 54.6 ~ 80.3%, respectively, and the chiroinositol-fed rats group, by 44.0 ~ 76.1% and 45.0 ~ 78.2%, respectively. This result demonstrates that pinitol and chiroinositol have distinct liver-protecting activity.

Example 4: Determination of SOD Activity in Liver

6 week-old, Sprague-Dawley rats (70 heads) were bred under the condition of 23±3°C, 55±15 % relative humidity, 12L/12D (a.m. 8 - p.m. 8) photoperiod and 150-300 Lux illumination. Fodder (Harlan, U.S.A.) and water were sterilized and fed to the rats.

The rats were divided into 7 groups and pinitol dissolved in 10 ml of water was orally administered to the rats of the experimental groups in an amount of 200-1000 mg/kg of rat body weight everyday for 4 days. The comparative group rats were treated with Silymarin (Sigma Chemical Co.) in an amount of 200 mg/kg, the normal and control groups were each treated with 10 ml of distilled water. On day 4, a mixture of carbon tetrachloride and soybean oil (1:1(v/v)) was injected intraperitoneally into the rats except the rats of the normal group in an amount of 0.5 ml/kg, 90 minutes after the administration of pinitol. 24 hours thereafter, SOD activity of the liver taken out from each rat was measured according to the method of Neoot et al., (Methods Enzymol., 186: 209-219 (1993)) and tested by student t-test. The result is shown in Table III.

Table III

|  | SOD activity (U/g Liver) | Relative Value (%) |
|---|---|---|
| Normal group | 209±51 | 90 |
| Control group | 233±29 | 100 |
| Comparative group | 191±25 | 82 |
| Experimental group 1 (pinitol 200 mg/kg) | 217±74 | 93 |
| Experimental group 2 (Pinitol 300 mg/kg) | 289±45 | 124 |
| Experimental group 3 (Pinitol 500 mg/kg) | 335±52 | 144 |
| Experimental group 4 (Pinitol 1000 mg/kg) | 315±38 | 135 |

As can be seen from Table III, SOD activities of the rats of the comparative group and experimental group 1 were lower than that of the control group by 18% and 7%, respectively, while those of experimental groups 2, 3 and 4 were higher by 24%, 44% and 35% than that of the control group. These results demonstrate that the administration of 300 mg/kg of pinitol raises SOD activity in the liver ($p < 0.05$).

Example 5: Measurement of Content of Glutathione in Liver

In order to induce diabetes in rats, streptozotocin (Sigma Chemical Co., USA) dissolved in 0.01M citrate buffer solution (pH 4.5) was injected into the abdominal cavity of 8 week-old Sprague-Dawley rats in an amount of 45 mg/kg of rat body weight. After 3 days, the blood glucose value of a venous blood sample taken out from the tail of each rat was measured and the rats having more than 251 ±7 mg/dl of blood glucose value deemed to the diabetic rats. The diabetic rats were divided into 3 groups, each of 12 - 15 rats, and pinitol or chiroinositol dissolved in 10 ml of water was orally administered to the rats of two groups (experimental groups 1 and 2) in an amount of 10 mg/kg of rat body weight everyday for 4 days. Rats of the normal group and control group (diabetic group) were each treated with 10 ml of distilled water. After fasting during 18 hours, the rats were anesthetized with ethyl ether, sacrificed, the liver was taken and washed with cold physiological saline. The glutathione content of the homogenized liver solution was measured according to the method of Ellman (Arch. Biochem. Biophy., 70-77 (1959)) and the result is shown in Table IV.

Table IV

|  | Glutathione content ($\mu$ mole/g) | Relative value |
|---|---|---|
| Normal group | 17.09$\pm$0.77 | 100 |
| Control group (Diabetic group) | 6.51$\pm$1.50 | 38.1 |
| Experimental group 1 (pinitol group) | 16.38$\pm$2.43 | 95.8 |
| Experimental group 1 (chiroinositol group) | 14.52$\pm$0.57 | 85.0 |

As can be seen from Table IV, the glutathione contents for the rats of the control group (diabetic group) was markedly lower than that of the normal group (38%), while those of the experimental group 1 (pinitol group) and experimental group 2 (chiroinositol group) were only slightly lower as compared to the normal group, exhibiting high values of 96% and 85%, respectively.

Example 6: Protective Activity for Liver

Pinitol was orally administered to adults (the average age: 51.8 years, man 7 and female 8) showing serum GOT or $\gamma$-GPT level higher than 50 IU/L at a daily dose of 600 mg for 2 months. Serum GOT, GPT and $\gamma$-GTP levels were determined before and after the administration. The results are shown in Table V.

Table V

|  | Before administration | After administration | Degree of decrease(%) |
|---|---|---|---|
| GOT(IU/L) | 57.4$\pm$10.6 | 31.4+3.0 | 45.3 |
| GPT(IU/L) | 89.6$\pm$22.3 | 43.2$\pm$8.1 | 51.8 |
| $\gamma$-GTP(IU/L) | 140.2$\pm$31.1 | 82.6$\pm$17.5 | 41.1 |

As can be see from Table V, serum GOT, GPT and $\gamma$-GTP levels decreased by 45.3%, 51.8% and 41.1%, respectively, after the administration.

Formulation Examples

The composition of the present invention can be used in preparing a pharmaceutical formulation by admixing the active ingredients with pharmaceutical excipients in various pharmaceutical forms according to any one of the conventional methods, as exemplified below.

<Formulation Example 1> Preparation of Powder

Pinitol     600mg
Lactose   1,400mg

The above ingredients were mixed thoroughly and then, filled and sealed in a sealed package to obtain a powder preparation.

<Formulation Example 2> Preparation of Tablet

Pinitol                200g

(continued)

| Corn Starch | 50mg |
| Lactose | 50mg |
| Steric Acid Magnesium | 2mg |

The above ingredients were mixed thoroughly and tabletted according to a conventional method to obtain a tablet preparation.

<Formulation Example 3> Preparation of Capsule

| Pinitol | 200mg |
| Corn Starch | 100mg |
| Lactose | 100mg |
| Steric Acid Magnesium | 2mg |

The above ingredients were mixed thoroughly and filled in a gelatin capsule according to a conventional method to obtain a capsule preparation.

<Formulation Example 4> Preparation of Injection Solution

| Pinitol | 200mg |
| Distilled water for injection | q.s. |
| pH adjuster | q.s. |

The above ingredients were dissolved in distilled water for injection, and adjusted to pH approximately 7.5. The resulting solution was filled in 2 mℓ of ample with distilled water for injection and sterilized according to a conventional method to obtain an injection preparation.

<Preparation of Health care beverage>

1-10 wt % of pinitol, 5-10 wt % of sugar, 0.05-0.3 wt % of citric acid, 0.005-0.02 wt % of caramel and 0.1-1 wt % of vitamin C were mixed and distilled water was added thereto to obtain a syrup. The syrup thus obtained was sterilized at 85-98 °C for 20-180 seconds, mixed with 4-fold volume of cool water, and 0.5 to 0.82 % of carbonic acid gas was added thereto, to obtain a carbonated beverage containing pinitol.
Also, pinitol was homogeneously mixed with liquid fructose (0.5%), oligosaccharides (2%), sugar (2%), saline (0.5%) and water (75%) and instantaneously sterilized to obtain a health beverage.

## Claims

1. Pinitol for use in a method of preventing or treating a liver disease in a mammal.

2. Pinitol for use according to claim 1, wherein pinitol enhances superoxide dismutase (SOD) activity.

3. Pinitol for use according to claim 1, wherein pinitol increases the glutathione level in the liver.

4. Pinitol for use according to claim 1, wherein the mammal is human.

5. Pinitol for use according to claim 1, wherein pinitol is administered to the mammal in the form of a composition containing same, said composition being selected from the group consisting of: a pharmaceutical composition, a food composition and a beverage composition.

6. An extract of a plant containing pinitol for use in a method of preventing or treating a liver disease in a mammal, wherein said plant is pine or carob.

7. Extract of a plant for use according to claim 6, wherein the plant extract containing pinitol enhances superoxide dismutase (SOD) activity.

8. Extract of a plant for use according to claim 6, wherein the plant extract containing pinitol increases the glutathione level in the liver.

9. Extract of a plant for use according to claim 6, wherein the mammal is human.

10. Extract of a plant for use according to claim 6, wherein the plant extract is a water extract or an organic solvent extract.

11. Extract of a plant for use according to claim 10, wherein the plant extract is prepared by adding 5 to 15-fold volume of water to a plant powder; extracting at 10 to 80°C for 1 to 24 hours; and filtering the extract thus obtained.

12. Extract of a plant for use according to claim 6, wherein the plant extract containing pinitol is administered to the mammal in the form of a composition containing the same, said composition being selected from the group consisting of: a pharmaceutical composition, a food composition and a beverage composition.

13. Pinitol or an extract of pine or carob comprising pinitol for use in a method of preventing or treating a liver disease in a mammal, which comprises administering an effective amount of pinitol or said extract of pine or carob thereto.

14. Pinitol or an extract of pine or carob for use according to claim 13, wherein the effective amount of pinitol is 0.1 to 100 mg/kg body weight/day.


**Patentansprüche**

1. Pinitol zur Verwendung in einem Verfahren zum Verhindern oder Behandeln einer Lebererkrankung in einem Säugetier.

2. Pinitol zur Verwendung nach Anspruch 1, wobei Pinitol die Superoxid-Dismutase (SOD)-Aktivität verstärkt.

3. Pinitol zur Verwendung nach Anspruch 1, wobei Pinitol die Glutathion-Konzentration in der Leber erhöht.

4. Pinitol zur Verwendung nach Anspruch 1, wobei das Säugetier ein Mensch ist.

5. Pinitol zur Verwendung nach Anspruch 1, wobei Pinitol an das Säugetier in der Form einer Pinitol-enthaltenden Zusammensetzung verabreicht wird, wobei die Zusammensetzung aus der Gruppe ausgewählt ist, bestehend aus: einer pharmazeutischen Zusammensetzung, einer Nahrungsmittelzusammensetzung und einer Getränkezusammensetzung.

6. Pflanzenextrakt, enthaltend Pinitol, zur Verwendung in einem Verfahren zum Verhindern oder Behandeln einer Lebererkrankung in einem Säugetier, wobei die Pflanze Pinie oder Kiefer ("pine") oder Johannisbrot ("carob") ist.

7. Pflanzenextrakt zur Verwendung nach Anspruch 6, wobei der Pinitol-enthaltende Pflanzenextrakt Superoxid-Dismutase (SOD)-Aktivität verstärkt.

8. Pflanzenextrakt zur Verwendung nach Anspruch 6, wobei der Pinitol-enthaltende Pflanzenextrakt die Glutathion-Konzentration in der Leber erhöht.

9. Pflanzenextrakt zur Verwendung nach Anspruch 6, wobei das Säugetier ein Mensch ist.

10. Pflanzenextrakt zur Verwendung nach Anspruch 6, wobei der Pflanzenextrakt ein Wasserextrakt oder ein organischer Lösungsmittelextrakt ist.

11. Pflanzenextrakt zur Verwendung nach Anspruch 10, wobei der Pflanzenextrakt hergestellt wird durch Zugabe des 5- bis 15-fachen Volumens an Wasser zu einem Pflanzenpulver; Extrahieren bei 10 bis 80°C für 1 bis 24 Stunden; und Filtern des so erhaltenen Extrakts.

**12.** Pflanzenextrakt zur Verwendung nach Anspruch 6, wobei der Pinitol-enthaltende Pflanzenextrakt dem Säugetier in der Form einer Pinitol-enthaltenden Zusammensetzung verabreicht wird, wobei die Zusammensetzung ausgewählt ist aus der Gruppe, bestehend aus: einer pharmazeutischen Zusammensetzung, einer Nahrungsmittelzusammensetzung und einer Getränkezusammensetzung.

**13.** Pinitol oder ein Pinien- oder Kiefern- oder Johannisbrot-Extrakt, umfassend Pinitol, zur Verwendung in einem Verfahren zum Verhindern oder Behandeln einer Lebererkrankung in einem Säugetier, wobei das Verfahren die Verabreichung einer wirksamen Menge an Pinitol oder des Pinien- oder Kiefern- oder Johannisbrot-Extrakts an das Säugetier umfasst.

**14.** Pinitol oder ein Pinien- oder Kiefern-Extrakt zur Verwendung nach Anspruch 13, wobei die wirksame Menge an Pinitol 0,1 bis 100 mg/kg Körpergewicht/Tag beträgt.

**Revendications**

**1.** Pinitol destiné à être utilisé dans un procédé de prévention ou de traitement d'une maladie hépatique chez un mammifère.

**2.** Pinitol destiné à être utilisé selon la revendication 1, dans lequel le pinitol accroît l'activité de la superoxyde dismutase (SOD).

**3.** Pinitol destiné à être utilisé selon la revendication 1, dans lequel le pinitol augmente le niveau de glutathion dans le foie.

**4.** Pinitol destiné à être utilisé selon la revendication 1, dans lequel le mammifère est un humain.

**5.** Pinitol destiné à être utilisé selon la revendication 1, dans lequel le pinitol est administré au mammifère sous la forme d'une composition le contenant, ladite composition étant choisie dans le groupe constitué par : une composition pharmaceutique, une composition alimentaire et une composition de boisson.

**6.** Extrait d'une plante contenant du pinitol destiné à être utilisé dans un procédé de traitement ou de prévention d'une maladie hépatique chez un mammifère, dans lequel ladite plante est le pin ou le caroubier.

**7.** Extrait d'une plante destiné à être utilisé selon la revendication 6, dans lequel l'extrait de plante contenant du pinitol accroît l'activité de la superoxyde dismutase (SOD).

**8.** Extrait d'une plante destiné à être utilisé selon la revendication 6, dans lequel l'extrait de plante contenant du pinitol augmente le niveau de glutathion dans le foie.

**9.** Extrait d'une plante destiné à être utilisé selon la revendication 6, dans lequel le mammifère est un humain.

**10.** Extrait d'une plante destiné à être utilisé selon la revendication 6, dans lequel l'extrait de plante est un extrait aqueux ou un extrait dans un solvant organique.

**11.** Extrait d'une plante destiné à être utilisé selon la revendication 10, dans lequel l'extrait de plante est préparé par ajout de 5 à 15 fois le volume d'eau à une poudre végétale ; par extraction à 10 à 80°C pendant 1 à 24 heures ; et par filtration de l'extrait ainsi obtenu.

**12.** Extrait d'une plante destiné à être utilisé selon la revendication 6, dans lequel l'extrait de plante contenant du pinitol est administré au mammifère sous la forme d'une composition le contenant, ladite composition étant choisie dans le groupe constitué par : une composition pharmaceutique, une composition alimentaire et une composition de boisson.

**13.** Pinitol ou extrait de pin ou de caroubier comprenant du pinitol destiné à être utilisé dans un procédé de prévention ou de traitement d'une maladie hépatique chez un mammifère, qui comprend l'administration d'une quantité efficace de pinitol ou dudit extrait de pin ou de caroubier à celui-ci.

14. Pinitol ou extrait de pin ou de caroubier destiné à être utilisé selon la revendication 13, dans lequel la quantité efficace de pinitol est de 0,1 à 100 mg/kg de poids corporel/jour.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5550166 A

### Non-patent literature cited in the description

- **ARTEEL GE.** *Gastroenterology,* 2003, vol. 124, 778-90
- **LOGUERCIO C ; FEDERICO A.** *Free Radic. Biol. Med.,* 2003, 1
- *FREE RADIC. BIOL. MED.,* vol. 34 (1), 1-10
- **MEHTA K et al.** *Nutr. Rev.,* 2002, vol. 60 (9), 289-93
- **GEBHARDT R.** *Planta Med.,* 2002, vol. 68 (4), 289-96
- **ADACHI M et al.** *Free Radic. Biol. Med.,* 2002, vol. 32 (6), 487-91
- **PAROLA M et al.** *J. Hepatol.,* 2001, vol. 35 (2), 297-306
- **NEOOT et al.** *Methods Enzymol.,* 1993, vol. 186, 209-219
- **ELLMAN.** *Arch. Biochem. Biophy.,* 1959, 70-77